(19) Europäisches Patentamt
European Patent Office
Office européen des brevets

(11) Veröffentlichungsnummer: **0 100 898**
**B1**

(12) **EUROPÄISCHE PATENTSCHRIFT**

(45) Veröffentlichungstag der Patentschrift: **13.05.87**

(51) Int. Cl.⁴: **C 07 D 293/12**, C 07 D 517/04, A 61 K 31/41

(21) Anmeldenummer: **83106873.9**

(22) Anmeldetag: **12.07.83**

(54) **Cycloalkylderivate von Benzisoselenazolonen, Verfahren zu ihrer Herstellung und diese enthaltende pharmazeutische Präparate.**

(30) Priorität: **14.07.82 DE 3226286**

(43) Veröffentlichungstag der Anmeldung: **22.02.84 Patentblatt 84/8**

(45) Bekanntmachung des Hinweises auf die Patenterteilung: **13.05.87 Patentblatt 87/20**

(84) Benannte Vertragsstaaten: **AT BE CH DE FR GB IT LI LU NL SE**

(56) Entgegenhaltungen:
**EP - A - 0 044 453**
**EP - A - 0 044 971**
**DE - A - 3 027 074**

**ARZNEIMITTELFORSCHUNG, Band 54, Nr. 12, Dezember 1964, Seiten 1301-1306, Editio Cantor, Aulendorf, DE., R. FISCHER et al.: "On benzisothiazolones: A series with a wide range of bacteriostatic and fungistatic activity"**

**Die Akte enthält technische Angaben, die nach dem Eingang der Anmeldung eingereicht wurden und die nicht in dieser Patentschrift enthalten sind.**

(73) Patentinhaber: **A. Nattermann & Cie. GmbH, Nattermannallee 1, D-5000 Köln 30 (DE)**

(72) Erfinder: **Welter, André, Dr., Reiherweg 11A, D-5024 Pulheim (DE)**
Erfinder: **Leyck, Sigurd, Dr., Am Quechenhauf 21, D-5024 Pulheim 2 (DE)**
Erfinder: **Etschenberg, Eugen, Dr., Hirseweg 10, D-5000 Köln 41 (DE)**

(74) Vertreter: **Redies, Bernd, Dr. rer. nat., COHAUSZ & FLORACK Patentanwaltsbüro Schumannstrasse 97 Postfach 14 01 47, D-4000 Düsseldorf 1 (DE)**

**EP 0 100 898 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue Benzisoselenazolone, Verfahren zu ihrer Herstellung sowie ihre Verwendung als Wirkstoffe in Arzneimitteln zur Behandlung von entzündlichen Erkrankungen des rheumatischen Formenkreises.

Die erfindungsgemässen Verbindungen entsprechen der allgemeinen Formel I

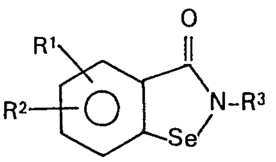

(I)

worin $R^1$ und $R^3$ gleich oder verschieden sein können und unabhängig voneinander Wasserstoff, Halogen, $C_1$-$C_4$-Alkyl, $C_1$-$C_4$-Alkoxy, Hydroxy, Trifluormethyl, Nitro, Di-($C_1$-$C_4$-alkyl)-amino oder $R^1$ und $R^2$ zusammen Methylendioxy bedeuten, während $R^3$ einen Cycloalkylrest mit 5-8 C-Atomen bedeutet, der gegebenenfalls eine Doppelbindung aufweist, oder einen Norbornyl-, Hexahydroindanyl-, Hexahydronaphthyl- oder Adamantylrest bedeutet.

Bevorzugt sind dabei Verbindungen, worin $R^1$ und $R^2$ gleich oder verschieden sein können und unabhängig voneinander Wasserstoff, Fluor, Chlor, Brom, Hydroxy, Methoxy, Methyl, Trifluormethyl, Nitro und/oder Methylendioxy bedeuten, während $R^3$ einen Cyclopentyl-, Cyclopentenyl-, Cyclohexyl-, Cyclohexenyl-, Cycloheptyl-, Cycloheptenyl-, Cyclooctyl-, Cyclooctenyl-, Norbornyl-, Hexahydroindanyl-, Hexahydronaphthyl- oder Adamantylrest bedeutet.

Besonders bevorzugt sind Verbindungen, worin $R^1$ und $R^2$ gleich oder verschieden sein können und unabhängig voneinander Wasserstoff, Chlor, Methyl, Methoxy, Nitro und/oder Methylendioxy bedeuten, während $R^3$ einen Cyclohexyl-, 2-Cyclohexenyl- oder 3-Cyclohexenylrest bedeutet.

Erfindungsgemässe Verbindungen sind beispielsweise:

2-Cyclopentyl-1,2-benzisoselenazol-3(2H)-on,
2-(3-Cyclopenten-1-yl)-1,2-benzisoselenazol-3(2H)-on,
2-Cyclohexyl-1,2-benzisoselenazol-3(2H)-on,
6-Methyl-2-cyclohexyl-1,2-benzisoselenazol-3(2H)-on,
6-Methoxy-2-cyclohexyl-1,2-benzisoselenazol-3(2H)-on,
6-Chlor-2-cyclohexyl-1,2-benzisoselenazol-3(2H)-on,
5-Nitro-2-cyclohexyl-1,2-benzisoselenazol-3(2H)-on,
5-Chlor-2-cyclohexyl-1,2-benzisoselenazol-3(2H)-on,
7-Methoxy-2-cyclohexyl-1,2-benzisoselenazol-3(2H)-on,
5,6-Methylendioxy-2-cyclohexyl-1,2-benzisoselenazol-3(2H)-on,
2-(2-Cyclohexen-1-yl)-1,2-benzisoselenazol-3(2H)-on,
2-(3-Cyclohexen-1-yl)-1,2-benzisoselenazol-3(2H)-on,
2-Cycloheptyl-1,2-benzisoselenazol-3(2H)-on,
2-(3-Cyclopenten-1-yl)-1,2-benzisoselenazol-3(2H)-on,
2-Cyclooctyl-1,2-benzisoselenazol-3(2H)-on,
2-(4-Cycloocten-1-yl)-1,2-benzisoselenazol-3(2H)-on,
2-(2-Norbornyl)-1,2-benzisoselenazol-3(2H)-on,
2-(2-Hexahydroindanyl)-1,2-benzisoselenazol-3(2H)-on,
2-(3-Hexahydroindanyl)-1,2-benzisoselenazol-3(2H)-on,
2-(1-Hexahydronaphthyl)-1,2-benzisoselenazol-3(2H)-on,
2-(2-(Hexahydronaphthyl)-1,2-benzisoselenazol-3(2H)-on,
2-(1-Adamantyl)-1,2-benzisoselenazol-3(2H)-on,
2-(2-Adamantyl)-1,2-benzisoselenazol-3(2H)-on.

Die erfindungsgemässen Benzisoselenazolone der allgemeinen Formel I können zur Behandlung zahlreicher Krankheiten verwendet werden, wie z.B. zur Prophylaxe und Therapie von Infektionskrankheiten, zur Stimulierung des Immunsystems oder bei Selenmangelkrankheiten, wie sie bei W. Kraus und P. Oehme, das Deut. Gesundheitswesen, 1979, 34 (37), 1713-1718 und 1979, 34 (37), 1769-1773, definiert werden.

Die Benzisoselenazolone der allgemeinen Formel I zeichnen sich jedoch besonders durch antiarteriosklerotische und entzündungshemmende Eigenschaften aus. Sie eignen sich besonders zur Therapie von rheumatischen Krankheiten, wie z.B. Arthrosen oder chronischer Polyarthritis, wobei die neuen Verbindungen sich durch eine sehr gute Verträglichkeit auszeichnen, da sie untoxisch sind und, im Gegensatz zu bekannten, entzündungshemmenden Therapeutika, keinerlei Ulkusbildung oder gastrointestinale Irritationen zeigen.

Die neuen Benzisoselenazolone der allgemeinen Formel I können in an sich bekannter Weise nach dem Verfahren gemäss Anspruch 9 erhalten werden. Dabei wird ein o-Chlorselenobenzoylchlorid der allgemeinen Formel II

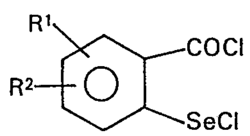

(II)

wobei $R^1$ und $R^2$ die in der allgemeinen Formel I angegebene Bedeutung haben mit einem Cycloalkylamin der allgemeinen Formel III

$R^3$-$NH_2$ (III)

wobei $R^3$ die in der allgemeinen Formel I angegebene Bedeutung hat, unter Ringschlussbedingungen zu Benzisoselenazolonen der allgemeinen Formel I umgesetzt.

Die Herstellung der entsprechenden o-Chlorselenobenzoylchloride erfolgt nach dem Verfahren

von A. Ruwet und M. Renson, Soc. Chim. Belg. 1966, 15, 157–163.

Als Ausgangsverbindungen der allgemeinen Formel II kommen zum Beispiel die folgenden Verbindungen in Betracht:
2-Chlorselenobenzoylchlorid,
2-Chlorseleno-4-chlorbenzoylchlorid,
2-Chlorseleno-4-methylbenzoylchlorid,
2-Chlorseleno-4-methoxybenzoylchlorid,
2-Chlorseleno-5-chlorbenzoylchlorid,
2-Chlorseleno-5-methoxybenzoylchlorid,
2-Chlorseleno-5-nitrobenzoylchlorid,
2-Chlorseleno-3-methoxybenzoylchlorid,
2-Chlorseleno-3,4-methylendioxybenzoylchlorid.

Als Ausgangsverbindungen III kommen z.B. in Frage:
Cyclopentylamin, 4-Amino-1-cyclopenten,
Cyclohexylamin, 3-Amino-1-cyclohexen,
4-Amino-1-cyclohexen, Cycloheptylamin,
5-Amino-1-cyclohepten, Cyclooctylamin,
5-Amino-1-cycloocten, 2-Norbornylamin,
2-Aminohexahydroindan,
3-Aminohexahydroindan,
1-Naphthylamin, 2-Naphthylamin,
1-Adamantylamin, 2-Adamantylamin.

Die Erfindung betrifft ebenfalls pharmazeutische Präparate, welche Verbindungen der allgemeinen Formel I enthalten. Bei den erfindungsgemässen pharmazeutischen Präparaten handelt es sich um solche zur enteralen, wie oralen oder rektalen sowie parenteralen Verabreichung, welche die pharmazeutischen Wirkstoffe allein oder zusammen mit einem üblichen pharmazeutisch anwendbaren Trägermaterial enthalten. Vorteilhafterweise liegt die pharmazeutische Zubereitung des Wirkstoffes in Form von Einzeldosen vor, die auf die gewünschte Verabreichung abgestimmt sind, wie z.B. Tabletten, Dragées, Kapseln, Suppositorien, Granulate, Lösungen, Emulsionen oder Suspensionen. Die Dosierung der Verbindungen liegt üblicherweise zwischen 10–1000 mg pro Tag, vorzugsweise zwischen 30–300 mg und kann ein- oder mehrmals, bevorzugt zwei- bis dreimal täglich, verabreicht werden.

Die Herstellung der erfindungsgemässen Verbindungen wird durch die folgenden Beispiele näher erläutert.

Beispiel 1

2-Cyclohexyl-1,2-benzisoselenazol-3(2H)-on

4,7 g (0,047 Mol) Cyclohexylamin, gelöst in 80 ml Tetrachlorkohlenstoff, werden unter Rühren und Eiskühlung (Temperatur 10°C) in einer Stickstoffatmosphäre langsam zu einer Lösung von 4 g (0,015 Mol) o-Chlorselenobenzoylchlorid in 60 ml Tetrachlorkohlenstoff zugetropft. Nach einer Stunde Rühren bei Raumtemperatur wird der ausgefallene Niederschlag abfiltriert, mit wenig Tetrachlorkohlenstoff, 0,5-normaler Salzsäure und Wasser gewaschen, getrocknet und aus Tetrachlorkohlenstoff und danach aus Toluol umkristallisiert.

Ausbeute: 3,1 g (70% d.Th.), F. 159–160°C
IR (in KBr): 1590 $cm^{-1}$
MS [m/e]: 281 (24,8%), 199 (100%), 184 (16,5%), 156 (14,2%)

Analog der Vorschrift von Beispiel 1 werden hergestellt:

Beispiel 2

2-(2-Cyclohexen-1-yl)-1,2-benzisoselenazol-3(2H)-on
Ausbeute: 67% d.Th., F. 142–143°C
IR (in KBr): 1585 $cm^{-1}$
MS [m/e]: 279 (42,3%), 199 (100%), 184 (45,2%), 156 (11,6%), 80 (96,9%)

Beispiel 3

2-(3-Cyclohexen-1-yl)-1,2-benzisoselenazol-3(2H)-on
Ausbeute: 66% d.Th., F. 169–171°C
IR (in KBr): 1610 $cm^{-1}$
MS [m/e]: 279 (17,5%), 225 (19,7%), 200 (100%), 184 (18,6%), 156 (27,3%), 80 (76,0%)

Analog der Vorschrift von Beispiel 1 werden hergestellt:
2-Cyclopentyl-1,2-benzisoselenazol-3(2H)-on,
6-Methyl-2-cyclohexyl-1,2-benzisoselenazol-3(2H)-on,
6-Methoxy-2-cyclohexyl-1,2-benzisoselenazol-3(2H)-on,
6-Chlor-2-cyclohexyl-1,2-benzisoselenazol-3(2H)-on,
5-Nitro-2-cyclohexyl-1,2-benzisoselenazol-3(2H)-on,
5-Chlor-2-cyclohexyl-1,2-benzisoselenazol-3(2H)-on,
7-Methoxy-2-cyclohexyl-1,2-benzisoselenazol-3(2H)-on,
5,6-Methylendioxy-2-cyclohexyl-1,2-benzisoselenazol-3(2H)-on,
2-Cycloheptyl-1,2-benzisoselenazol-3(2H)-on,
2-(3-Cyclopenten-1-yl)-1,2-benzisoselenazol-3(2H)-on,
2-Cyclooctyl-1,2-benzisoselenazol-3(2H)-on,
2-(4-Cycloocten-1-yl)-1,2-benzisoselenazol-3(2H)-on,
2-(2-Norbornyl)-1,2-benzisoselenazol-3(2H)-on,
2-(2-Hexahydroindinanyl)-1,2-benzisoselenazol-3(2H)-on,
2-(3-Hexahydroindanyl)-1,2-benzisoselenazol-3(2H)-on,
2-(1-Hexahydronaphthyl)-1,2-benzisoselenazol-3(2H)-on,
2-(2-Hexahydronaphthyl)-1,2-benzisoselenazol-3(2H)-on,
2-(1-Adamantyl)-1,2-benzisoselenazol-3(2H)-on,
2-(2-Adamantyl)-1,2-benzisoselenazol-3(2H)-on.

**Patentansprüche für die Vertragsstaaten: BE, CH, DE, FR, GB, IT, LI, LU, NL, SE**

1. Benzisoselenazolone der allgemeinen Formel I

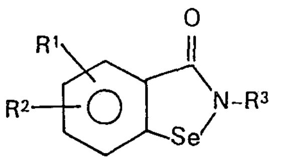

(I)

worin $R^1$ und $R^2$ gleich oder verschieden sein können und unabhängig voneinander Wasserstoff, Halogen, $C_1$ bis $C_4$-Alkyl, $C_1$ bis $C_4$-Alkoxy, Hydroxy, Trifluormethyl, Nitro, Di-($C_1$ bis $C_4$-alkyl)-amino oder $R^1$ und $R^2$ zusammen Methylendioxy bedeuten, während $R^3$ einen Cycloalkylrest mit 5 bis 8 C-Atomen, der gegebenenfalls eine Doppelbindung aufweist, oder einen Norbornyl-, Hexahydroindanyl-, Hexahydronaphthyl- oder Adamantylrest bedeutet.

2. Benzisoselenazolone gemäss Anspruch 1, worin $R^1$ und $R^2$ gleich oder verschieden sein können und unabhängig voneinander Wasserstoff, Fluor, Chlor, Brom, Hydroxy, Methoxy, Methyl, Trifluormethyl, Nitro oder Methylendioxy bedeuten, während $R^3$ einen Cyclopentyl-, Cyclopentenyl-, Cyclohexyl-, Cyclohexenyl-, Cycloheptyl-, Cycloheptenyl-, Cyclooctyl-, Cyclooctenyl-, Norbornyl-, Hexahydroindanyl-, Hexahydronaphthyl- oder Adamantylrest bedeutet.

3. Benzisoselenazolone gemäss Anspruch 1, worin $R^1$ und $R^2$ gleich oder verschieden sein können und unabhängig voneinander Wasserstoff, Chlor, Methyl, Methoxy, Nitro oder Methylendioxy bedeuten, während $R^3$ einen Cyclohexyl-, 2-Cyclohexenyl- oder 3-Cyclohexenylrest bedeutet.

4. 2-Cyclohexyl-1,2-benzisoselenazol-3(2H)-on.

5. 6-Chlor-2-cyclohexyl-1,2-benzisoselenazol-3(2H)-on.

6. 6-Methoxy-2-cyclohexyl-1,2-benzisoselenazol-3(2H)-on.

7. 2-(2-Cyclohexen-1-yl)-1,2-benzisoselenazol-3(2H)-on.

8. 2-(3-Cyclohexen-1-yl)-1,2-benzisoselenazol-3(2H)-on.

9. Verfahren zur Herstellung von Verbindungen gemäss den Ansprüchen 1-8, dadurch gekennzeichnet, dass man in an sich bekannter Weise o-Chlorselenobenzoylchloride der allgemeinen Formel II

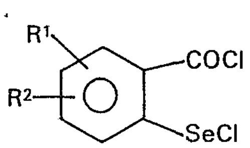

(II)

worin $R^1$ und $R^2$ die gleiche Bedeutung wie in der allgemeinen Formel I haben, mit einem Cycloalkylamin der allgemeinen Formel III

$R^3$–$NH_2$ (III)

wobei $R^3$ die in der allgemeinen Formel I angegebene Bedeutung hat, unter Ringschlussbedingungen zu den Benzisoselenazolonen der allgemeinen Formel I umsetzt.

10. Pharmazeutische Präparate, dadurch gekennzeichnet, dass sie eine Verbindung der allgemeinen Formel I gemäss den Ansprüchen 1-8 als Wirkstoffe im Gemisch mit üblichen pharmazeutischen Hilfs- und Trägerstoffen enthalten.

**Patentansprüche für den Vertragsstaat: AT**

1. Verfahren zur Herstellung von Benzisoselenazolonen der allgemeinen Formel I

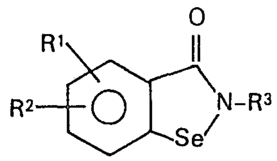

(I)

worin $R^1$ und $R^2$ gleich oder verschieden sein können und unabhängig voneinander Wasserstoff, Halogen, $C_1$–$C_4$-Alkyl, $C_1$–$C_4$-Alkoxy, Hydroxy, Trifluormethyl, Nitro, Di-($C_1$–$C_4$-alkyl)-amino oder $R^1$ und $R^2$ zusammen Methylendioxy bedeuten, während $R^3$ einen Cycloalkylrest mit 5–8 C-Atomen, der gegebenenfalls eine Doppelbindung aufweist, oder einen Norbornyl-, Hexahydroindanyl-, Hexahydronaphthyl- oder Adamantylrest bedeutet, dadurch gekennzeichnet, dass man in an sich bekannter Weise o-Chlorselenobenzoylchloride der allgemeinen Formel II

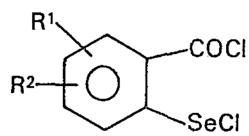

(II)

worin $R^1$ und $R^2$ die gleiche Bedeutung wie in der allgemeinen Formel I haben, mit einem Cycloalkylamin der allgemeinen Formel III

$R^3$–$NH_2$ (III)

wobei $R^3$ die in der allgemeinen Formel I angegebene Bedeutung hat, unter Ringschlussbedingungen zu den Benzisoselenazolonen der allgemeinen Formel I umsetzt.

2. Verfahren gemäss Anspruch 1, dadurch gekennzeichnet, dass man von Verbindungen der Formel II, worin $R^1$ und $R^2$ gleich oder verschieden sein können und unabhängig voneinander Wasserstoff, Fluor, Chlor, Brom, Hydroxy, Methoxy, Methyl, Trifluormethyl, Nitro oder Methylendioxy bedeuten, und von Verbindungen der Formel III, worin $R^3$ einen Cyclopentyl-, Cyclopentenyl-, Cyclohexyl-, Cyclohexenyl-, Cycloheptyl-, Cycloheptenyl-, Cyclooctyl-, Cyclooctenyl-, Norbornyl-, Hexahydroindanyl-, Hexahydronaphthyl- oder Adamantylrest bedeutet, ausgeht.

3. Verfahren gemäss Anspruch 1, dadurch gekennzeichnet, dass man von Verbindungen der Formel II, worin $R^1$ und $R^2$ gleich oder verschieden sein können und unabhängig voneinander Wasserstoff, Chlor, Methyl, Methoxy, Nitro oder Methylendioxy bedeuten, und von Verbindungen der

Formel III, worin $R^3$ einen Cyclohexyl-, 2-Cyclohexenyl- oder 3-Cyclohexenylrest bedeutet, ausgeht.

**Claims for the contracting State: AT**

1. Process for producing of Benzisoselenazolones of the general formula I

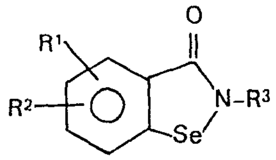

(I)

in which $R^1$ and $R^2$ can be identical or different and, independently of one another, denote hydrogen, halogen, $C_1$-$C_4$-alkyl, $C_1$-$C_4$-alkoxy, hydroxy, trifluoromethyl, nitro, di-($C_1$-$C_4$-alkyl)-amino, or $R^1$ and $R^2$ together denote methylenedioxy, while $R^3$ denotes a cycloalkyl radical having 5 to 8 carbon atoms which optionally has a double bond, or denotes a norbornyl, hexahydroindanyl, hexahydronaphthyl or adamantyl radical, characterised in that o-chloroselenobenzoyl chlorides in a manner known per se of the general formula II

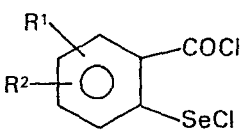

(II)

in which $R^1$ and $R^2$ have the same meaning as in general formula I, are reacted with a cycloalkylamine of the general formula III

$R^3$–$NH_2$ (III)

$R^3$ having the meaning indicated in general formula I, under conditions for ring closure to give the benzisoselenazolones of the general formula I.

2. Process according to claim 1, wherein it is started of compounds of formula II in which $R^1$ and $R^2$ can be identical or different and, independently of one another, denote hydrogen, fluorine, chlorine, bromine, hydroxy, methoxy, methyl, trifluoromethyl, nitro or methylenedioxy, and compounds of formula III in which $R^3$ denotes a cyclopentyl, cyclopentenyl, cyclohexyl, cyclohexenyl, cycloheptyl, cycloheptenyl, cyclooctyl, cyclooctenyl, norbornyl, hexahydroindanyl, hexahydronaphthyl or adamantyl radical.

3. Process according to claim 1, wherein it is started of compounds of formula II in which $R^1$ and $R^2$ can be identical or different, and, independently of one another, denote hydrogen, chlorine, methyl, methoxy, nitro or methylenedioxy, and compounds of formula III in which $R^3$ denotes a cyclohexyl, 2-cyclohexenyl or 3-cyclohexenyl radical.

**Claims for the contracting States: BE, CH, DE, FR, GB, IT, LI, LU, NL, SE**

1. Benzisoselenazolones of the general formula I

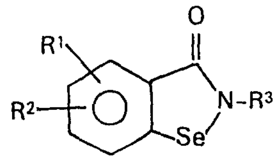

(I)

in which $R^1$ and $R^2$ can be identical or different and, independently of one another, denote hydrogen, halogen, $C_1$-$C_4$-alkyl, $C_1$-$C_4$-alkoxy, hydroxy, trifluoromethyl, nitro, di-($C_1$-$C_4$-alkyl)-amino, or $R^1$ and $R^2$ together denote methylenedioxy, while $R^3$ denotes a cycloalkyl radical having 5 to 8 carbon atoms which optionally has a double bond, or denotes a norbornyl, hexahydroindanyl, hexahydronaphthyl or adamantyl radical.

2. Benzisoselenazolones according to claim 1, in which $R^1$ and $R^2$ can be identical or different and, independently of one another, denote hydrogen, fluorine, chlorine, bromine, hydroxy, methoxy, methyl, trifluoromethyl, nitro or methylenedioxy, while $R^3$ denotes a cyclopentyl, cyclopentenyl, cyclohexyl, cyclohexenyl, cycloheptyl, cycloheptenyl, cyclooctyl, cyclooctenyl, norbornyl, hexahydroindanyl, hexahydronaphthyl or adamantyl radical.

3. Benzisoselenazolones according to claim 1, in which $R^1$ and $R^2$ can be identical or different, and, independently of one another, denote hydrogen, chlorine, methyl, methoxy, nitro or methylenedioxy, while $R^3$ denotes a cyclohexyl, 2-cyclohexenyl or 3-cyclohexenyl radical.

4. 2-Cyclohexyl-1,2-benzisoselenazol-3(2H)-one.

5. 6-Chloro-2-cyclohexyl-1,2-benzisoselenazol-3(2H)-one.

6. 6-Methoxy-2-cyclohexyl-1,2-benzisoselenazol-3(2H)-one.

7. 2-(2-Cyclohexen-1-yl)-1,2-benzisoselenazol-3(2H)-one.

8. 2-(3-Cyclohexen-1-yl)-1,2-benzisoselenazol-3(2H)-one.

9. Process for the preparation of compounds according to claims 1 to 8, characterised in that o-chloroselenobenzoyl chlorides of the general formula II

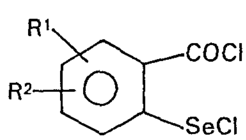

(II)

in which $R^1$ and $R^2$ have the same meaning as in general formula I, are reacted, in a manner known per se, with a cycloalkylamine of the general formula III

$R^3$–$NH_2$ (III)

$R^3$ having the meaning indicated in general formula I, under conditions for ring closure to give the benzisoselenazolones of the general formula I.

10. Pharmaceutical products, characterised in that they contain a compound of the general formula I according to claims 1 to 8 as the active ingredients in a mixture with customary pharmaceutical auxiliaries and vehicles.

**Revendications pour les Etats contractants: BE, CH, DE, FR, GB, IT, LI, LU, NL, SE**

1. Benzisosélénazolone de formule générale I

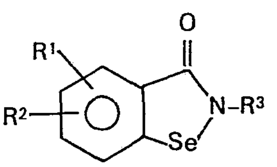

(I)

où $R^1$ et $R^2$ peuvent être identiques ou différents et représentent indépendamment l'un de l'autre un hydrogène, un halogène, un alcoyle en $C_1$ à $C_4$, un alcoxy en $C_1$ à $C_4$, un hydroxy, un trifluorométhyle, un nitro, un di-(alcoyle en $C_1$ à $C_4$)-amino ou $R^1$ et $R^2$ représentent ensemble un méthylènedioxy, tandis que $R^3$ représente un radical cycloalcoyle en $C_5$ à $C_8$, qui présente éventuellement une double liaison, ou un radical norbornyle, hexahydroindanyle, hexahydronaphtyle ou adamantyle.

2. Benzisosélénazolone selon la revendication 1, où $R^1$ et $R^2$ peuvent être identiques ou différents et représentent indépendamment l'un de l'autre un hydrogène, un fluor, un chlore, un brome, un hydroxy, un méthoxy, un méthyle, un trifluorométhyle, un nitro ou un méthylènedioxy, tandis que $R^3$ représente un radical cyclopentyle, cyclopentényle, cyclohexyle, cyclohexényle, cycloheptyle, cycloheptényle, cyclooctyle, cyclooctényle, norbornyle, hexahydroindanyle, hexahydronaphtyle ou adamantyle.

3. Benzisosélénazolone selon la revendication 1, où $R^1$ et $R^2$ peuvent être identiques ou différents et représentent indépendamment l'un de l'autre un hydrogène, un chlore, un méthyle, un méthoxy, un nitro ou un méthylènedioxy, tandis que $R^3$ représente un radical cyclohexyle, 2-cyclohexényle ou 3-cyclohexényle.

4. 2-cyclohexyl-1,2-benzisosélénazol-3(2H)-one.

5. 6-chloro-2-cyclohexyl-1,2-benzisosélénazol-3(2H)-one.

6. 6-méthoxy-2-cyclohexyl-1,2-benzisosélénazol-3(2H)-one.

7. 2-(2-cyclohexen-1-yl)-1,2-benzisosélénazol-3(2H)-one.

8. 2-(3-cyclohexen-1-yl)-1,2-benzisosélénazol-3(2H)-one.

9. Procédé de préparation de composés selon les revendications 1–8, caractérisé en ce qu'on fait réagir de façon connue des chlorures de o-chlorosélénobenzoyle de formule générale II

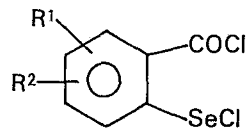

(II)

où $R^1$ et $R^2$ ont la même signification que dans la formule générale I, avec une cycloalcoylamine de formule générale III

$R^3$–$NH_2$ (III)

où $R^3$ a la signification indiquée dans la formule générale I, dans des conditions de cyclisation pour donner les benzisosélénazolones de formule générale I.

10. Préparations pharmaceutiques caractérisées en ce qu'elles contiennent un composé de formule générale I selon les revendications 1–8 comme substances actives mélangées à des additifs et supports pharmaceutiques habituels.

**Revendications pour l'Etat contractant: AT**

1. Procédé de préparation de benzisosélénazolone de formule générale I

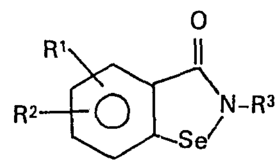

(I)

où $R^1$ et $R^2$ peuvent être identiques ou différents et représentent indépendamment l'un de l'autre un hydrogène, un halogène, un alcoyle en $C_1$ à $C_4$, un alcoxy en $C_1$ à $C_4$, un hydroxy, un trifluorométhyle, un nitro, un di-(alcoyle en $C_1$ à $C_4$)-amino ou bien où $R^1$ et $R^2$ représentent ensemble un méthylènedioxy, tandis que $R^3$ représente un radical cycloalcoyle en $C_5$ à $C_8$, qui présente éventuellement une double liaison, ou un radical norbornyl, hexahydroindanyle, hexahydronaphtyle ou adamantyle, caractérisé en ce qu'on fait réagir de façon connue des chlorures de o-chlorosélénobenzoyle de formule générale II

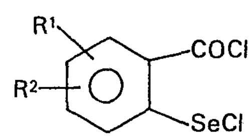

(II)

où $R^1$ et $R^2$ ont la même signification que dans la formule générale I, avec une cycloalcoylamine de formule générale III

$R^3$–$NH_2$ (III)

où $R^3$ a la signification donnée dans la formule générale I, dans des conditions de cyclisation pour donner les benzisosélénazolones de formule générale I.

2. Procédé selon la revendication 1, caractérisé en ce qu'on part de composés de formule II, où $R^1$ et $R^2$ peuvent être identiques ou différents et représentent indépendamment l'un de l'autre un hydrogène, un fluor, un chlore, un brome, un hydroxy, un méthoxy, un méthyle, un trifluorométhyle, un nitro ou un méthylènedioxy, et de composés de formule III, où $R^3$ représente un radical cyclopentyle, cyclopentényle, cyclohexyle, cyclohexényle, cycloheptyle, cycloheptényle, cyclooctyle, cyclooctényle, norbornyle, hexahydroindanyle, hexahydronaphtyle ou adamantyle.

3. Procédé selon la revendication 1, caractérisé en ce qu'on part de composés de formule II, où $R^1$ et $R^2$ peuvent être identiques ou différents et représentent indépendamment l'un de l'autre un hydrogène, un chlore, un méthyle, un méthoxy, un nitro ou un méthylènedioxy, et de composés de formule III, où $R^3$ représente un radical cyclohexyle, 2-cyclohexényle ou 3-cyclohexényle.